# EUROPÄISCHE PATENTSCHRIFT

(11) **EP 4 091 712 B1**
(45) Veröffentlichungstag und Bekanntmachung des Hinweises auf die Patenterteilung: **15.05.2024**
(21) Anmeldenummer: 21174300.0
(22) Anmeldetag: 18.05.2021
(51) Int. Cl.: B01J 31/40

(54) **VERFAHREN ZUR REGENERIERUNG EINES KATALYSATORS FÜR DIE HYDROFORMYLIERUNG VON OLEFINEN IN DER GASPHASE**
METHOD FOR REGENERATING A CATALYST FOR THE HYDROFORMYLATION OF OLEFINS IN THE GAS PHASE
PROCÉDÉ DE RÉGÉNÉRATION D'UN CATALYSEUR POUR L'HYDROFORMYLATION D'OLÉFINES EN PHASE GAZEUSE

(43) Veröffentlichungstag der Anmeldung: 23.11.2022
(73) Patentinhaber: Evonik Oxeno GmbH & Co. KG, 45772 Marl (DE)
(72) Erfinder: FRANKE, Robert, 45772 Marl (DE); ARSENJUK, Linda, 44147 Dortmund (DE); SCHÜLLER, Jessika, 56767 Gunderath (DE); STENGER, Frank, 63755 Alzenau (DE); FLEISCHER, Vinzenz, 45770 Marl (DE); KRISTEN, Marc Oliver, 45721 Haltern am See (DE)
(74) Vertreter: Evonik Patent Association

(56) Entgegenhaltungen:
- EP-A1- 3 632 885
- EP-A1- 3 632 888

## Beschreibung

Gegenstand der Erfindung ist ein einfaches Verfahren zur Regenerierung eines Hydroformylierungskatalysator, der aus einem auf einem Support aus einem porösen keramischen Material heterogenisierten Katalysatorsystem besteht. Gegenstand ist auch ein Verfahren zum Anfahren der Hydroformylierungsreaktion nach der erfindungsgemäßen Regenerierung.

Die Hydroformylierung ist mit einer jährlichen globalen Produktionskapazität von mehreren Millionen Tonnen eine der bedeutendsten Reaktionen in der großtechnischen Chemie. Dabei werden Alkene (Olefine) mit einer Mischung aus Kohlenmonoxid und Wasserstoff (auch: Synthesegas oder Syngas) unter Verwendung eines Katalysators zu Aldehyden umgewandelt, die wichtige und wertvolle Zwischenprodukte bei der Herstellung von chemischen Massenprodukten wie Alkoholen, Estern oder Weichmachern sind.

Die Hydroformylierung wird im großtechnischen Maßstab ausschließlich homogen katalysiert durchgeführt. Die löslichen Übergangsmetallkatalysatorsysteme basieren üblicherweise auf Cobalt oder Rhodium, welches oft mit Phosphor-haltigen Liganden, bspw. Phosphinen oder Phosphiten, für die Hydroformylierung von eher kurzkettigen Olefinen eingesetzt wird.

Die Probleme bei den bekannten Verfahren sind vielfältig, die insbesondere damit verknüpft sind, dass sowohl Rhodium als auch Cobalt und deren Verbindungen vergleichsweise teuer sind. Es wird ein hoher energetischer und verfahrenstechnischer Aufwand betrieben, um Katalysatorverluste während des Hydroformylierungsprozesses weitestgehend zu vermeiden, beispielsweise durch teils sehr aufwändige Katalysatorrecyclingschritte. Zudem werden Produktaufreinigungsschritte aufwendiger, um sicherzustellen, dass möglichst keine Katalysatorrückstände im Produkt verbleiben.

Weitere Probleme bei den bekannten homogen katalysierten Verfahren sind die Stabilität der Liganden, die die Bedingungen der Hydroformylierung, wie Temperatur, Druck, pH-Wert, usw., überstehen müssen, und der Verbrauch von dem verwendeten Lösemittel während des Prozesses, der durch Nachdosieren ausgeglichen werden muss.

Um die vorgenannten Probleme bei der homogen katalysierten Hydroformylierung zu umgehen, sind Hydroformylierungsverfahren entwickelt worden, bei denen das Katalysatorsystem heterogenisiert wird, insbesondere durch Immobilisierung auf einem Trägermaterial (vgl. einleitende Diskussion in der WO 2015/028284 A1). Vor kurzer Zeit wurden auch auf Monolithen heterogenisierte Systeme entwickelt, die beispielsweise in den Patentanmeldungen EP 3 632 885 A1, EP 3 744 707 A1, EP 3 532 886 A1, EP 3 736 258 A1, EP 3 632 888 A1, EP 3 632 887 A1 oder EP 3 632 889 A1. Die Begriffe Heterogenisierung und Immobilisierung sind demnach so zu verstehen, dass der Katalysator durch Ausbildung eines dünnen Flüssigkeitsfilms mithilfe einer ionischen Flüssigkeit auf der Oberfläche und/oder in den Poren eines festen Trägermaterials immobilisiert wird und keine Reaktionslösung im klassischen Sinne vorliegt, in der der Katalysator homogen gelöst ist.

Das Problem bei den oben genannten immobilisierten oder heterogenisierten Systemen ist jedoch, dass nach einer gewissen Laufzeit eine Abnahme der Katalysatoraktivität und damit eine Verringerung des Umsatzes und der Selektivität beobachtet werden kann. Dies kann auf verschiedene Effekte zurückzuführen sein, beispielsweise eine Kondensation der Produkte in den Poren und entsprechende Folgereaktionen wie Aldolkondensationen, oder die Bildung von Wasser, die zu einer Desaktivierung der Liganden führen kann, die Bildung von Nebenprodukten und/oder die Flutung der Poren, wodurch der Katalysator ausgetragen werden kann.

Die Aufgabe der vorliegenden Erfindung bestand deshalb darin, ein einfaches Verfahren zur Regenerierung von Hydroformylierungskatalysatoren, die aus einem auf einem Support aus einem porösen keramischen Material heterogenisierten Katalysatorsystem bereitzustellen.

Diese Aufgabe wird durch das erfindungsgemäße Verfahren gemäß Anspruch 1 gelöst. Dabei wird der Katalysator in einem Behälter mit einer Lösung aus einem Lösemittel und einem Phosphor-enthaltenden Liganden in Kontakt gebracht. Der Gegenstand der vorliegenden Erfindung ist deshalb ein Verfahren zur Regenerierung eines in einem Behälter befindlichen Hydroformylierungskatalysators, der aus einem heterogenisierten Katalysatorsystem besteht,
wobei das Katalysatorsystem ein Metall aus der 8. oder 9. Gruppe des Periodensystems der Elemente und mindestens einen organischen Phosphor-enthaltenden Liganden umfasst und heterogenisiert auf einem Support vorliegt, wobei der Support aus einem porösen keramischen Material besteht und in Form eines Granulats oder in Form eines Monolithen vorliegt,
wobei das Verfahren zumindest die folgenden Schritte umfasst:
   a) Befüllen des Behälters mit einer Lösung, die aus dem Phosphor-enthaltenden Liganden und einem Lösungsmittel besteht, und Stehenlassen für mindestens eine Stunde,
   b) Ablassen der Lösung aus dem Behälter.

Der Vorteil des erfindungsgemäßen Verfahrens ist, dass es sehr einfach und kostengünstig zu realisieren ist. Im Gegensatz zu bekannten Verfahren muss die Lösung zum Regenerieren zwar den Liganden enthalten, nicht jedoch das Metall aus der 8. oder 9. Gruppe des Periodensystems der Elemente. Die Herstellung der Lösung ist somit weniger aufwendig. Da die geeigneten Metalle für das Katalysatorsystem außerdem recht teuer sind, können diese Kosten beim erfindungsgemäßen Verfahren eingespart werden.

Es ist nach dem erfindungsgemäßen Verfahren überraschenderweise möglich den Hydroformylierungskatalysator zu regenerieren, in dem dieser in einer Lösung, die aus dem Phosphor-enthaltenden Liganden und einem Lösungsmittel besteht, für mindestens eine Stunde stehengelassen wird. In einer bevorzugten Ausführungsform der vorliegenden Erfindung wird der Hydroformylierungskatalysator für mindestens 12 Stunden, besonders bevorzugt für mindestens 24 Stunden in der Lösung stehengelassen. Grundsätzlich kann der Hydroformylierungskatalysator durchaus für eine längere Dauer in der Lösung stehengelassen werden. Aufgrund der Tatsache, dass der Katalysator aber schnellstmöglich wieder in der Produktion eingesetzt werden soll, bietet es sich an den Katalysator für nicht mehr als 72 Stunden in der Lösung stehen zu lassen.

Der Behälter, in dem sich der Hydroformylierungskatalysator befindet, kann grundsätzlich jede Art von Behälter sein. Vorzugsweise ist der Behälter aber ein geschlossener Behälter, der zumindest je einen Eingang und einen Ausgang aufweist. Besonders bevorzugt ist der Behälter ein Reaktor, in dem eine Hydroformylierung unter Verwendung des Hydroformylierungskatalysators stattfinden kann. In einer besonders bevorzugten Ausführungsform der vorliegenden Erfindung findet das Verfahren in-situ in dem Reaktor statt, in dem sich der Hydroformylierungskatalysator schon bei der vorangegangen Hydroformylierung befunden hat. Dadurch kann der Hydroformylierungskatalysator im Reaktor verbleiben und muss nicht erst ausgebaut und in einen anderen Behälter eingefüllt werden. Trotzdem kann es aus anlagen- oder prozesstechnischen Gründen vorteilhaft sein, wenn der Katalysator ausgebaut wird und in einem anderen Behälter regeneriert wird, die Regenerierung also ex-situ stattfindet.

Bei der in-situ Regenerierung kann es vorteilhaft sein, wenn mehrere, d. h. mindestens zwei parallele Reaktoren vorliegen. Dann kann ein sogenannter Swing-Cycle durchgeführt werden. Das bedeutet, dass mindestens einer der Reaktoren in Betrieb ist und in dem die gewünschte Hydroformylierung durchgeführt wird, während mindestens ein anderer Reaktor regeneriert wird. Sobald die Regenerierung abgeschlossen ist kann der jeweilige Reaktor ebenfalls in Betrieb genommen werden oder bis zum Einsatz in Wartestellung gehalten werden.

Bevor die Regenerierung durchgeführt wird, egal ob sie in-situ oder ex-situ stattfindet, werden zunächst die Eduktströme für die Hydroformylierung, also die zu hydroformylierende Substanz und das Synthesegas, abgestellt. Anschließend kann der Reaktor, in dem sich dann noch der Hydroformylierungskatalysator befindet, mit einem Inertgas gespült werden. Dadurch können Reste der Edukte ausgetrieben werden. Außerdem wird der Reaktor durch das Austreiben von Edukte inertisiert. Es ist weiterhin bevorzugt, dass der Volumenstrom des beim Spülen benutzten Inertgases pro Stunde dem 1-fachen bis 100-fachen des Volumens des Behälters bzw. des Reaktors beträgt. Als Inertgase für das Spülen eignen sich alle bekannten Inertgase. Vorzugsweise wird das Inertgas aus der Gruppe, bestehend aus Stickstoff, Helium, Neon, CO₂ und Argon, ausgewählt wird. Besonders bevorzugt ist Stickstoff als Inertgas. Wird der Reaktor gespült, ist es bevorzugt, dass der Reaktor während des Spülens auf Umgebungstemperatur abkühlt.

Im ersten Schritt des erfindungsgemäßen Verfahrens (Schritt a)) wird der Behälter, in dem sich der Hydroformylierungskatalysator dann befindet (in-situ oder ex-situ), mit der Lösung zum Regenerieren befüllt. Vorzugsweise wird das Befüllen bei Raumtemperatur (20 bis 25 °C) und Normaldruck (ca. 1 bar) durchgeführt. Weiterhin bevorzugt findet das Befüllen unter Inertgasatmosphäre statt, insbesondere in einen vorher inertisierten Behälter bzw. Reaktor. Als Inertgase für das Befüllen eignen sich alle bekannten Inertgase. Vorzugsweise wird das Inertgas aus der Gruppe, bestehend aus Stickstoff, Helium, Neon, CO₂ und Argon, ausgewählt wird. Besonders bevorzugt ist Stickstoff als Inertgas.

Nachdem der Hydroformylierungskatalysator für mindestens eine Stunde, vorzugsweise für mindestens 12 Stunden, besonders bevorzugt für mindestens 24 Stunden in dem Behälter bzw. Reaktor (ex-situ oder in-situ) stehen gelassen worden ist, wird die Lösung aus dem Behälter bzw. Reaktor in Schritt b) abgelassen. Das kann entweder mittels einer geeigneten Apparatur, beispielsweise einer Pumpe, geschehen oder ohne eine solche Apparatur, d. h. hydrostatisch, erfolgen. Vorzugsweise erfolgt das Ablassen der Lösung in Schritt b) hydrostatisch.

Es kann sein, dass nach dem Ablassen der Lösung aus dem Behälter bzw. dem Reaktor in Schritt b) des erfindungsgemäßen Verfahrens noch Lösemittelreste vorhanden sind. Um die Lösemittelreste zu entfernen oder um zu verhindern, dass Lösemittelreste im Behälter bzw. Reaktor zurückbleiben, kann während des Ablassens in Schritt b) oder nach dem Ablassen der Lösung die Temperatur erhöht und/oder der Druck im Behälter bzw. Reaktor reduziert werden, um noch vorhandenes Lösemittel abzudampfen. In einer alternativen Ausführungsform der vorliegenden Erfindung ist das Lösemittel das Reaktionsprodukt der vorherigen Hydroformylierung, also der dabei entstandene Aldehyd. In einem solchen Fall reicht es, wenn das Lösemittel abgelassen wird. Ein zusätzliches Abdampfen ist dann nicht unbedingt erforderlich.

Sofern während des Ablassens in Schritt b) oder nach dem Ablassen der Lösung die Temperatur erhöht und/oder der Druck im Behälter bzw. Reaktor reduziert wird, sind die exakten Werte für Temperatur und Druck in weiten Bereichen variabel. Dabei kommt es auf das Lösemittel und seine Siedetemperatur in Abhängigkeit vom Druck an. Schließlich sieden Substanzen bei geringerer Temperatur, wenn der Druck geringer ist. Insofern ist die Angabe von speziellen Temperaturen schwierig. In einer bevorzugten Ausführungsform wird die Temperatur in Schritt b) auf mindestens 80 °C, vorzugsweise mindestens 90 °C erhöht. Es ist weiterhin bevorzugt, dass der Reaktor beim Erhöhen der Temperatur und/oder beim Reduzieren des Drucks mit einem Inertgas durchströmt wird. Als Inertgase eignen sich dafür alle bekannten Inertgase. Vorzugsweise wird das Inertgas aus der Gruppe, bestehend aus Stickstoff, Helium, Neon, CO₂ und Argon, ausgewählt wird. Besonders bevorzugt ist Stickstoff als Inertgas. Es ist weiterhin bevorzugt, dass die Inertgase, die für das Spülen, das Befüllen (Schritt a) und das Ablassen (Schritt b)) identisch sind, um den präparativen Aufwand zu verringern.

Die Lösung, die für das Regenerieren verwendet wird, besteht erfindungsgemäß aus dem Phosphor-enthaltenden Liganden des eingesetzten Hydroformylierungskatalysators und einem Lösungsmittel. Als Lösemittel kommen alle Substanzen in Betracht, die in der Lage sind den Phosphor-enthaltenden Liganden zu lösen. Als Lösemittel kommen beispielsweise Dichlormethan, THF, Pentanol, Propanal, Propanol oder Pentanal in Betracht. Die Konzentration des organischen Phosphor-enthaltende Liganden kann in Abhängigkeit von Löslichkeitsgrenze in dem gewählten Lösemittel variieren. Die Konzentration des organischen Phosphor-enthaltenden Liganden beträgt vorzugsweise zwischen 1 und 70 g/L, vorzugsweise zwischen 5 und 40 g/L.

Der organische Phosphor-enthaltende Ligand des erfindungsgemäßen Katalysatorsystems kann jeder für die Hydroformylierung geeignete Ligand sein. Entsprechende Liganden sind in der Fachliteratur beschrieben und dem Fachmann bekannt. Vorzugsweise weist der organische Phosphor-enthaltende Ligand die allgemeine Formel (I)

R'-A-R"-A-R‴ (I)

auf, wobei R', R" und R‴ jeweils organische Reste sind, mit der Maßgabe das R' und R‴ nicht identisch sind, und beide A jeweils eine brückende -O-P(-O)₂-Gruppe sind, wobei zwei der drei Sauerstoffatome -O-jeweils an Rest R' und den Rest R‴ gebunden sind. Die organischen Reste R', R" und R‴ enthalten vorzugsweise keine endständige Trialkoxysilangruppe.

In einer bevorzugten Ausführungsform sind R', R" und R‴ in der Verbindung der Formel (VI), vorzugsweise ausgewählt aus substituierten oder unsubstituierten 1,1'-Biphenyl-, 1,1'-Binaphthyl- und ortho-Phenylgruppen, insbesondere aus substituierten oder unsubstituierten 1,1'-Biphenylgruppen, mit der Maßgabe das R' und R‴ nicht identisch sind. Besonders bevorzugt weisen die substituierten 1,1'-Biphenylgruppen in 3,3'- und/oder 5,5'-Stellung des 1,1'-Biphenylgrundkörpers eine Alkylgruppe und/oder eine Alkoxygruppe auf, insbesondere eine C1-C4-Alkylgruppe, besonders bevorzugt eine tert.-Butyl- und/oder Methylgruppe und/oder bevorzugt einen C1-C5-Alkoxgruppe, besonders bevorzugt eine Methoxygruppe. Beispiele für entsprechende Verbindungen ist der Ligand Biphephos, der z. B. in der DE 10 2011 002 640 A1 beschrieben wird, oder Liganden, die in der WO 2014/056733 A1 oder der EP 3 318 569 A1 beschrieben sind.

Das erfindungsgemäße Katalysatorsystem umfasst weiterhin ein Metall aus der 8. oder 9. Gruppe des Periodensystems der Elemente. Typische Metalle für die Hydroformylierung sind dem Fachmann bekannt. Das Metall aus der 8. oder 9. Gruppe des Periodensystems der Elemente wird vorzugweise aus der Gruppe, bestehend aus Eisen, Ruthenium, Iridium, Ruthenium, Cobalt oder Rhodium, ausgewählt. Besonders bevorzugt sind Cobalt und Rhodium.

Das vorgenannte Katalysatorsystem liegt erfindungsgemäß heterogenisiert auf einem Support aus einem porösen keramischen Material vor. Im Sinne der vorliegenden Erfindung ist der Ausdruck "heterogenisiert auf einem Support" so zu verstehen, dass das Katalysatorsystem durch Ausbildung eines dünnen, festen oder flüssigen Films mithilfe des Stabilisators und/oder optional der ionischen Flüssigkeit auf der inneren und/oder äußeren Oberfläche eines festen Trägermaterials immobilisiert wird. Der Film kann auch bei Raumtemperatur fest und bei Reaktionsbedingungen flüssig sein.

Das poröse Supportmaterial ist vorzugsweise ausgewählt aus der Gruppe, bestehend aus einer nitridischen Keramik, einer carbidischen Keramik, einer silicidischen Keramik und Mischungen davon, beispielsweise carbonitridische Materialien.

Die nitridische Keramik ist vorzugsweise ausgewählt aus Siliciumnitrid, Bornitrid, Aluminiumnitrid und Mischungen davon. Die carbidische Keramik ist vorzugsweise ausgewählt aus Siliciumcarbid, Borcarbid, Wolframcarbid oder Mischungen davon. Denkbar sind auch Mischungen aus carbidischen und nitridischen Keramik, die sogenannten Carbonitride. Die silicidische Keramik ist vorzugsweise Molybdändisilicid. Der Support gemäß der vorliegenden Erfindung, auf den das Katalysatorsystem aufgebracht wird, besteht vorzugsweise aus einer carbidischen Keramik, besonders bevorzugt aus Siliciumcarbid.

Der Support liegt erfindungsgemäß entweder in Form eines Monolithen oder in Form eines Granulats vor. Liegt der Support als Monolith vor, ist der Begriff Monolith so zu verstehen, dass der Support aus einem Block aus dem keramischen Material besteht, also ein dreidimensionales Objekt ist. Der Block kann sowohl einstückig ausgebildet sein als auch aus mehreren, also mindestens zwei Einzelteilen bestehen, die zu dem Block zusammengefügt werden können und/oder miteinander fest oder lösbar verbunden sind.

Liegt der Support als Granulat vor, besteht der Support aus kleinteiligen Partikeln. Die Größe der Partikel ist variabel und beispielsweise abhängig von der Reaktionsführung. Bei schnellen Reaktionen bieten sich eher kleine Partikel an, während bei langsam verlaufenden Reaktionen auch größere Partikeln eingesetzt werden können. Es ist bevorzugt, dass der mittlere Partikeldurchmesser (d50) des Supports, wenn er in Form des Granulats vorliegt, im Bereich von 0,01 mm bis 70 mm, vorzugsweise 0,03 bis 60 mm, besonders bevorzugt von 0,05 mm bis 50 mm beträgt. Der mittlere Partikeldurchmesser kann mittels bildgebender Verfahren ermittelt werden, insbesondere durch die in den Normen ISO 13322-1 (Stand: 2004-12-01) und ISO 13322-2 (Stand: 2006-11-01) genannten Verfahren ermittelt werden. Die Herstellung des Granulats kann nach dem Fachmann bekannten Verfahren erfolgen. Beispielsweise könnte es dadurch erfolgen, dass ein Monolith aus dem carbidischen, nitridischen, silicidischen Material oder Mischungen davon mechanisch zerkleinert wird, beispielsweise mit einem Backenbrecher, und die Partikelgröße des erhaltenen Bruchgranulats mittels Sieben eingestellt wird.

Darüber hinaus ist der Support, egal ob der in Form eines Monolithen oder in Form eines Granulats vorliegt, porös, d. h. der Support weist Poren auf. Der Porendurchmesser liegt vorzugsweise im Bereich von 0,9 nm bis 30 µm, vorzugsweise im Bereich von 10 nm bis 25 µm und besonders bevorzugt im Bereich von 70 nm bis 20 µm. Der Porendurchmesser kann mittels Stickstoffadsorption oder Quecksilber-Porosimetrie gemäß DIN 66133 (Stand: 1993-06) bestimmt werden. In einer bevorzugten Ausführungsform weist der Support zumindest teilweise durchgängige Poren auf, die sich von einer Oberfläche zu einer anderen Oberfläche hin erstrecken. Möglich ist auch, dass mehrere Poren miteinander verbunden sind und so insgesamt eine einzige durchgängige Pore bilden.

Die Herstellung des Supports aus einem porösen keramischen Material, auf dem das Katalysatorsystem heterogenisiert vorliegt, erfolgt wie nachfolgend beschrieben: Auf den bereitgestellten Support aus dem keramischen Material wird zusätzlich ein sogenannter Washcoat aufgetragen, welcher bezogen auf das keramische Material des Supports aus dem gleichen oder einem unterschiedlichen Keramikmaterial, vorzugsweise Siliciumoxid. Der Washcoat selbst kann porös oder unporös sein, vorzugsweise ist der Washcoat unporös. Die Partikelgröße des Washcoats beträgt vorzugsweise 5 nm bis 3 µm, vorzugsweise 7 nm bis 700 nm. Der Washcoat wird verwendet, um die gewünschte Porengröße einzubringen oder zu generieren und/oder, um die Oberfläche des Supports zu erhöhen. Der Auftrag des Washcoats kann insbesondere mittels Eintauchens (Dipcoating) in eine Washcoat-Lösung, die das Keramikmaterial des Washcoats, ggf. auch als Precursor, enthält, erfolgen. Die Menge das auf dem Support befindlichen Washcoats beträgt ≤ 20 Gew.-%, vorzugsweise ≤ 15 Gew.-%, besonders bevorzugt ≤ 10 Gew.-% bezogen auf die Gesamtmenge des Supports.

Anschließend kann das Katalysatorsystem aufgetragen werden, beispielsweise durch Imprägnierung, wie dies in den Patentanmeldungen EP 3 632 885 A1 oder WO 2020/070052 Q1 beschrieben worden ist.

Das Katalysatorsystem kann nebem dem Liganden und dem Metall noch weitere Substanzen enthalten. Der Vorteil des erfindungsgemäßen Regenerierverfahrens ist, dass die Lösung zum Regenerieren keine der nachfolgend genannten Substanzen enthalten muss. Es ist erfindungsgemäß bevorzugt, dass das Katalysatorsystem zusätzlich einen Stabilisator enthält. der Stabilisator für das erfindungsgemäße Katalysatorsystem ist vorzugsweise eine organische Aminverbindung, besonders bevorzugt eine organische Aminverbindung, die mindestens eine 2,2,6,6-Tetramethylpiperidineinheit nach Formel (II) enthält:

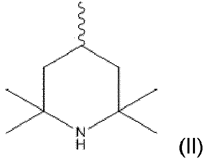

In einer besonders bevorzugten Ausführungsform der vorliegenden Erfindung ist der Stabilisator ausgewählt aus der Gruppe, bestehend aus den Verbindungen der nachfolgenden Formeln (II.1), (II.2), (II.3), (II.4), (II.5), (II.6), (II.7) und (II.8). 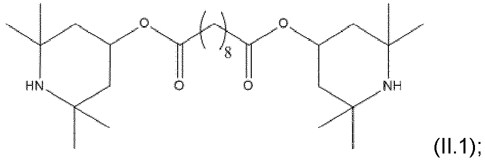 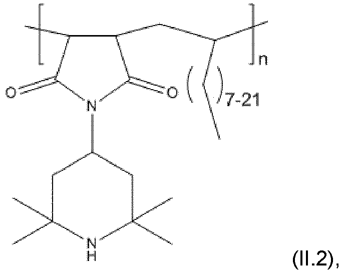 wobei n einer ganzen Zahl von 1 bis 20 entspricht; 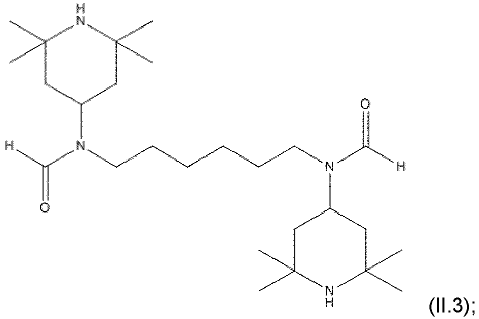 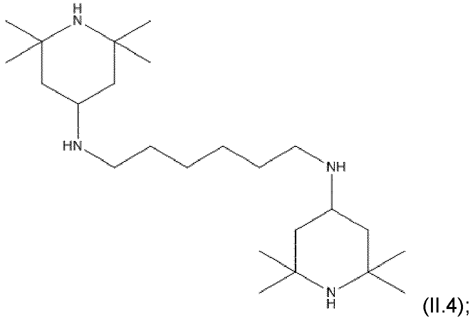 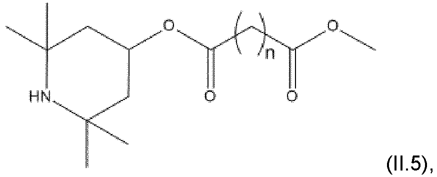 wobei n einer ganzen Zahl von 1 bis 12 entspricht; 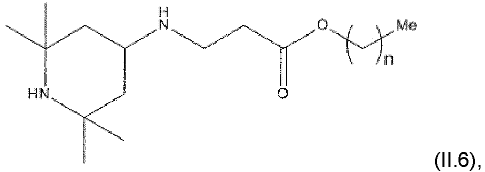 wobei n einer ganzen Zahl von 1 bis 17 entspricht; 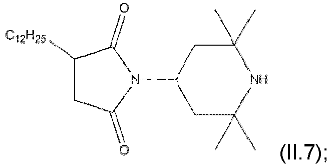 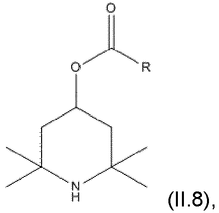 wobei R einer C6- bis C20-Alkylgruppe entspricht.

Das Katalysatorsystem kann außerdem eine ionische Flüssigkeit umfassen. Es ist jedoch bevorzugt, wenn das Katalysatorsystem keine ionische Flüssigkeit enthält.

Ist das Regenerierverfahren abgeschlossen kann die Reaktion, also die Hydroformylierung, wieder gestartet werden. Wurde die Regenerierung ex-situ durchgeführt, muss das Katalysatorsystem natürlich zunächst in den Hydroformylierungsreaktor eingefüllt werden. Bei einer in-situ Regenerierung liegt das Katalysatorsystem dort schon vor. Nach dem Regenerieren werden der Reaktor bzw. die Reaktion angefahren. Das entsprechende Verfahren zeichnet sich dadurch aus, dass ein gasförmiges Einsatzgemisch, welches zu hydroformylierende C2- bis C8-Olefine umfasst, zusammen mit einem Synthesegasgemisch in den Reaktor eingeleitet wird, und die Zusammensetzung des Einsatzgemisches und/oder die Zusammensetzung des Synthesegasgemisches bei konstantem Volumenstrom in mehreren Schritten dadurch variiert wird, dass der Anteil an zu hydroformylierenden C2- bis C8-Olefinen im Einsatzgemisch und/oder der Anteil von Synthesegas im Synthesegasgemisch schrittweise erhöht wird, mit der Maßgabe, dass ein maximaler Umsatz der eingesetzten C2- bis C8-Olefine von 40 bis 90% während des gesamten Anfahrens nicht überschritten wird.

Mit diesem Verfahren kann eine schonende Aktivierung und eine Abfederung der maximalen Startaktivität des Hydroformylierungskatalysators erreicht werden. Das führt zu einer verlängerten Lebenszeit des Katalysators, sowie zur partiellen oder vollständigen Verhinderung der Flüssigphasenbildung, die eine Desaktivierung oder das Auswaschen des Katalysatorsystems zur Folge haben kann.

Als Einsatzgemisch für das erfindungsgemäße Verfahren können alle Gemische eingesetzt werden, die C2- bis C8-Olefine, vorzugsweise C2- bis C5-Olefine, insbesondere Ethen, Propen, 1-Buten, 2-Buten, Isobuten, 1-Penten oder 2-Penten, als Edukte umfassen. Die Menge an Olefinen in den Einsatzgemischen sollte verständlicherweise hoch genug sein, um eine Hydroformylierungsreaktion wirtschaftlich betreiben zu können. Dazu gehören insbesondere technische Gemische der petrochemischen Industrie, wie beispielsweise Raffinatströme (Raffinat I, II oder III) oder Rohbutan. Rohbutan umfasst gemäß der vorliegenden Erfindung 5 bis 40 Gew.-% Butene, vorzugsweise 20 bis 40 Gew.-% Butene (die Butene setzen sich zusammen aus 1 bis 20 Gew.-% 1-Buten und 80 bis 99 Gew.-% 2-Buten) und 60 bis 95 Gew.-% Butane, vorzugsweise 60 bis 80 Gew.-% Butane.

Das Synthesegasgemisch gemäß der vorliegenden Erfindung besteht aus Synthesegas für die Hydroformylierung, enthält also Wasserstoff und Kohlenmonoxid, vorzugsweise in einem molaren Verhältnis von 60 : 40 bis 40 : 60, insbesondere 50 : 50, und optional geringe Mengen an Verunreinigungen. Das Synthesegas kann durch bekannte Verfahren erzeugt und bereitgestellt werden.

In der Anfahrprozedur ist vorgesehen, dass entweder der Anteil des Synthesegases im Synthesegasgemisch oder der Anteil der Olefine im Einsatzgemisch durch schrittweise Erhöhung variiert werden. Die Variation der Anteile von Synthesegas und Olefinen können auch zusätzlich zueinander erfolgen, wobei die Anzahl der Schritte bei der Erhöhung der Anteile auch unterschiedlich voneinander sein kann. Zu Beginn sollte der Anteil des Synthesegases und/oder der Anteil der Olefine geringer sein, als im üblichen Einsatz bei der Hydroformylierung und dann sukzessive gesteigert werden bis nach vollständigem Anfahren und Übergang zu Hydroformylierung die finale(n) Zusammensetzung(en) erreicht ist.

Die Erhöhung des Anteils der Olefine am Einsatzgemisch und/oder der Anteil von Synthesegas am Synthesegasgemisch erfolgt in mehreren Schritten, also in mindestens zwei Schritten. In einer bevorzugten Ausführungsform erfolgt die Erhöhung Anteils der Olefine am Einsatzgemisch und/oder der Anteil von Synthesegas am Synthesegasgemisch in mindestens drei Schritten, besonders bevorzugt in mindestens 4 Schritten.

Um eine Variation des Anteils der Olefine im Einsatzgemisch und/oder des Anteils an Synthesegas im Synthesegasgemisch erreichen zu können, kann anfänglich ein Inertgas zum Einsatzgemisch und/oder zum Synthesegasgemisch hinzugefügt werden, um das Einsatzgemisch und/oder das Synthesegasgemisch zu verdünnen.

Im ersten Schritt wird vorzugsweise so viel Inertgas zum Einsatzgemisch und/oder zum Synthesegasgemisch hinzugefügt wird, dass der Anteil des Inertgases im Einsatzgemisch und/oder im Synthesegasgemisch im Bereich von 70% bis 90% liegt. Der Anteil des Inertgases im Einsatzgemisch und/oder im Synthesegasgemisch wird dann in dem folgenden Schritt oder in den folgenden Schritten sukzessive reduziert. In dem letzten Schritt des Anfahrens beträgt der Anteil des Inertgases im Einsatzgemisch und/oder im Synthesegasgemisch dann nur noch 10% bis 30%. Als Inertgase eignen sich dafür alle bekannten Inertgase. Vorzugsweise wird das Inertgas aus der Gruppe, bestehend aus Stickstoff, Helium, Neon, CO₂ und Argon, ausgewählt wird. Besonders bevorzugt ist Stickstoff als Inertgas.

Nach dem letzten Schritt wird die Zugabe des Inertgases vollständig gestoppt. Einsatzgemisch und/oder Synthesegasgemisch werden dann nicht mehr verdünnt, sondern werden in ihrer normalen, unverdünnten Zusammensetzung eingesetzt. Dadurch ist die Anfahrprozedur beendet und der Reaktor ist wieder im Normalbetrieb.

### Beispiel:

In einem Rohrreaktor wurden ein aus einem heterogenisierten Katalysatorsystem bestehender Hydroformylierungskatalysator (Rh, Biphephos, Bis(2,2,6,6-tetramethyl-4-piperidyl)sebacat (Stabilisator) auf einem Support aus SiC, Support als Bruchgranulat mit 3 mm Partikelgröße (mittlere Fraktionsgröße)), vorgelegt. Für die Hydroformylierung wurde der Reaktor mit einem gasförmigen C4-Feed (Raffinat III) und Synthesegas kontinuierlich durchströmt. Die Hydroformylierung erfolgte bei einem Druck von 10 bar und einer Temperatur von 130 °C. Während der Reaktion wurde die n/iso-Selektivität des erzeugten Aldehyds bestimmt (Online-GC am Reaktorausgang zur Messung der Produktzusammensetzung). Es konnte festgestellt werden, dass die n/iso-Selektivität mit der Zeit abnimmt. Nach ca. 750 h wurde die Reaktion gestoppt und eine Regenerierung durchgeführt.

Die Vorgehensweise bei der Regenerierung war wie folgt:
- Stopp der Edukt-Ströme;
- Spülen des Trägers mit Stickstoff während des Abkühlens der Anlage auf Raumtemperatur;
- Fluten des Reaktorinnenraums & Träger mit Ligandlösung (Zusammensetzung Ligandlösung: Biphephos in Dichlormethan (ca. 67g/L));
- Halten der Lösung im Reaktor für ca. 48 Stunden;
- Ablassen der Lösung;
- Durchströmen des Reaktors mit Stickstoff (ca. 5g/h) und Erhöhung der Temperatur auf 120°C;
- Anfahren der Anlage (in Stufen, C4-Feed jeweils mit N2 verdünnt, wie in der EP 3 362 887 A1 beschrieben).

Nach dem Anfahren befand sich die Anlage wieder im Normalbetrieb, d. h. unter den oben genannten Hydroformylierungsbedingungen, die auch vor der Regenerierung vorlagen. Die n/iso-Selektivität konnte im Vergleich mit dem Wert vor der Regenerierung vollständig wiederhergestellt werden (s. nachfolgende Tabelle 1):

**Tabelle 1: Selektivitäten der untersuchten Reaktion**

| | |
|---|---|
| Selektivität zu Versuchsbeginn | 95% |
| n-iso- Selektivität vor Behandlung mit Ligandlösung | 72% |
| (Raff III, 10bar, 130°C) | |
| n-iso-Selektivität nach Behandlung mit Ligandlösung | 96% |
| (Raff III, 10bar, 130°C) | |

## Patentansprüche

1. Verfahren zur Regenerierung eines in einem Behälter befindlichen Hydroformylierungskatalysators, der aus einem heterogenisierten Katalysatorsystem besteht,
wobei das Katalysatorsystem ein Metall aus der 8. oder 9. Gruppe des Periodensystems der Elemente und mindestens einen organischen Phosphor-enthaltenden Liganden umfasst und heterogenisiert auf einem Support vorliegt, wobei der Support aus einem porösen keramischen Material besteht und in Form eines Granulats oder in Form eines Monolithen vorliegt,
wobei das Verfahren zumindest die folgenden Schritte umfasst:
a) Befüllen des Behälters mit einer Lösung, die aus dem Phosphor-enthaltenden Liganden und einem Lösungsmittel besteht, und Stehenlassen für mindestens eine Stunde,
b) Ablassen der Lösung aus dem Behälter.

2. Verfahren nach Anspruch 1, wobei die Lösung im Reaktor in Schritt a) mindestens 12 Stunden, vorzugsweise mindestens 24 Stunden gehalten wird.

3. Verfahren nach Anspruch 1 oder 2, wobei das Verfahren in-situ in dem Reaktor erfolgt, in dem sich der Hydroformylierungskatalysator befindet.

4. Verfahren nach einem der vorhergehenden Ansprüche, wobei das Lösungsmittel der Lösung Dichlormethan, THF, Pentanol, Propanal, Propanol oder Pentanal ist.

5. Verfahren nach einem der vorhergehenden Ansprüche, wobei das Ablassen der Lösung in Schritt b) hydrostatisch erfolgt.

6. Verfahren nach einem der vorhergehenden Ansprüche, wobei Schritt a) bei Umgebungstemperatur durchgeführt wird.

7. Verfahren nach einem der vorhergehenden Ansprüche, wobei der Reaktor beim Erhöhen der Temperatur in Schritt b) mit einem Inertgas durchströmt wird.

8. Verfahren nach Anspruch 7, wobei das Inertgas Stickstoff, Helium, Neon, CO₂ oder Argon ist.

9. Verfahren nach einem der vorhergehenden Ansprüche, wobei der Support aus einem porösen keramischen Material besteht, welches aus der Gruppe, bestehend aus einer Silikatkeramik, einer oxidischen Keramik, einer nitridischen Keramik, einer carbidischen Keramik, einer silicidischen Keramik und Mischungen davon, ausgewählt wird.

10. Verfahren nach einem der vorherigen Ansprüche, wobei auf den Support ein Washcoat aus, bezogen auf das keramische Material des Supports, dem gleichen oder einem anderen keramischen Material aufgetragen ist.

11. Verfahren nach Anspruch 10, wobei die Menge das auf dem Support befindlichen Washcoats ≤ 20 Gew.-% beträgt, bezogen auf die Gesamtmenge des Supports.

12. Verfahren nach einem der vorhergehenden Ansprüche, wobei das Katalysatorsystem zusätzlich einen Stabilisator umfasst.

13. Verfahren nach einem der vorhergehenden Ansprüche, wobei der Reaktor nach dem Regenerieren des Hydroformylierungskatalysators angefahren wird, wobei das Verfahren **dadurch gekennzeichnet ist, dass**
ein gasförmiges Einsatzgemisch, welches zu hydroformylierende C2- bis C8-Olefine umfasst, zusammen mit einem Synthesegasgemisch in den Reaktor eingeleitet wird, und
die Zusammensetzung des Einsatzgemisches und/oder die Zusammensetzung des Synthesegasgemisches bei konstantem Volumenstrom in mehreren Schritten dadurch variiert wird, dass der Anteil an zu hydroformylierenden C2- bis C8-Olefinen im Einsatzgemisch und/oder der Anteil von Synthesegas im Synthesegasgemisch schrittweise erhöht wird,
mit der Maßgabe, dass ein maximaler Umsatz der eingesetzten C2- bis C8-Olefine von 40 bis 90% während des gesamten Anfahrens nicht überschritten wird.

14. Verfahren nach Anspruch 13, wobei dem Einsatzgemisch und/oder dem Synthesegasgemisch ein Inertgas zur Verringerung des Anteils an C2- bis C8-Olefinen und/oder zur Verringerung des Anteils an Synthesegas hinzugefügt und die Zugabe des Inertgases entsprechend der schrittweisen Erhöhung des Anteils an C2- bis C8-Olefinen und/oder des Anteils an Synthesegas schrittweise reduziert wird.

## Claims

1. Process for regenerating a hydroformylation catalyst consisting of a heterogenized catalyst system, in a vessel,
wherein the catalyst system comprises a metal of Group 8 or 9 of the Periodic Table of the Elements and at least one organic phosphorus-containing ligand and is present heterogenized on a support, wherein the support consists of a porous ceramic material and is in the form of granules or in the form of a monolith,
wherein the process comprises at least the following steps:
a) filling the vessel with a solution consisting of the phosphorus-containing ligand and a solvent, and allowing it to stand for at least one hour,
b) discharging the solution from the vessel.

2. Process according to Claim 1, wherein the solution in the reactor in step a) is kept for at least 12 hours, preferably at least 24 hours.

3. Process according to Claim 1 or 2, wherein the process is carried out *in situ* in the reactor in which the hydroformylation catalyst is present.

4. Process according to any of the preceding claims, wherein the solvent of the solution is dichloromethane, THF, pentanol, propanal, propanol or pentanal.

5. Process according to any of the preceding claims, wherein the solution in step b) is discharged hydrostatically.

6. Process according to any of the preceding claims, wherein step a) is carried out at ambient temperature.

7. Process according to any of the preceding claims, wherein the reactor is flushed with an inert gas when raising the temperature in step b).

8. Process according to Claim 7, wherein the inert gas is nitrogen, helium, neon, CO₂ or argon.

9. Process according to any of the preceding claims, wherein the support consists of a porous ceramic material which is selected from the group consisting of a silicate ceramic, an oxidic ceramic, a nitridic ceramic, a carbidic ceramic, a silicidic ceramic and mixtures thereof.

10. Process according to any of the preceding claims, wherein a washcoat is applied to the support which, based on the ceramic material of the support, is composed of the same or another ceramic material.

11. Process according to Claim 10, wherein the amount of washcoat on the support is ≤ 20% by weight, based on the total amount of the support.

12. Process according to any of the preceding claims, wherein the catalyst system additionally comprises a stabilizer.

13. Process according to any of the preceding claims, wherein the reactor is started up after regeneration of the hydroformylation catalyst, wherein the process is **characterized in that**
a gaseous feedstock mixture comprising C2- to C8-olefins to be hydroformylated together with a synthesis gas mixture is introduced into the reactor, and
the composition of the feedstock mixture and/or the composition of the synthesis gas mixture is varied in two or more steps at a constant volume flow rate in that the proportion of C2- to C8-olefins to be hydroformylated in the feedstock mixture and/or the proportion of synthesis gas in the synthesis gas mixture is increased in a stepwise manner,
with the proviso that a maximum conversion of the C2-to C8-olefins used of 40 to 90% during the entire start-up is not exceeded.

14. Process according to Claim 13, wherein an inert gas is added to the feedstock mixture and/or to the synthesis gas mixture to reduce the proportion of C2- to C8-olefins and/or to reduce the proportion of synthesis gas and the addition of the inert gas is reduced in a stepwise manner corresponding to the stepwise increase of the proportion of C2- to C8-olefins and/or of the proportion of synthesis gas.

## Revendications

1. Procédé de régénération d'un catalyseur d'hydroformylation, qui est constitué d'un système catalyseur hétérogénisé, se trouvant dans un récipient,
dans lequel le système catalyseur comprend un métal du 8e ou du 9e groupe du système périodique des éléments et au moins un ligand organique contenant du phosphore et se présente sous forme hétérogénisé sur un support, le support étant constitué d'un matériau céramique poreux et se présentant sous forme d'un granulat ou sous forme d'un monolithe,
le procédé comprenant au moins les étapes suivantes :
a) remplissage du récipient avec une solution qui est constituée du ligand contenant du phosphore et d'un solvant, et abandon au repos pendant au moins une heure,
b) élimination de la solution du récipient.

2. Procédé selon la revendication 1, dans lequel la solution est maintenue dans le réacteur de l'étape a) pendant au moins 12 heures, de préférence au moins 24 heures.

3. Procédé selon la revendication 1 ou 2, le procédé étant réalisé *in situ* dans le réacteur dans lequel se trouve le réacteur d'hydroformylation.

4. Procédé selon l'une des revendications précédentes, dans lequel le solvant de la solution est le dichlorométhane, le THF, le pentanol, le propanal, le propanol ou le pentanal.

5. Procédé selon l'une des revendications précédentes, dans lequel l'élimination de la solution dans l'étape b) a lieu par voie hydrostatique.

6. Procédé selon l'une des revendications précédentes, dans lequel l'étape a) est mise en œuvre à la température ambiante.

7. Procédé selon l'une des revendications précédentes, dans lequel le réacteur est, lors de l'élévation de la température dans l'étape b), traversé par un gaz inerte.

8. Procédé selon la revendication 7, dans lequel le gaz inerte est l'azote, l'hélium, le néon, le CO₂ ou l'argon.

9. Procédé selon l'une des revendications précédentes, dans lequel le support est constitué d'un matériau céramique poreux, qui est choisi dans le groupe consistant en une céramique de silicate, une céramique à base d'oxyde, une céramique à base de nitrure, une céramique à base de carbure, une céramique à base de siliciure ou des mélanges de celles-ci.

10. Procédé selon l'une des revendications précédentes, dans lequel une couche lavis, en un matériau céramique identique ou différent au matériau céramique du support, est appliquée sur le support.

11. Procédé selon la revendication 10, dans lequel la quantité de la couche lavis se trouvant sur le support est < 20 % en poids par rapport à la quantité totale du support.

12. Procédé selon l'une des revendications précédentes, dans lequel le système catalyseur comprend en outre un stabilisant.

13. Procédé selon l'une des revendications précédentes, dans lequel le réacteur est démarré après la régénération du catalyseur d'hydroformylation, le procédé étant **caractérisé en ce que**
on introduit dans le réacteur un mélange d'entrée gazeux, qui comprend des oléfines en C2 à C8 à hydroformyler, en même temps qu'un mélange de gaz de synthèse, et
on fait varier la composition du mélange d'entrée et/ou la composition du mélange de gaz de synthèse, pour un débit volumique constant, en plusieurs étapes, en augmentant pas à pas la proportion d'oléfines en C2 à C8 à hydroformyler dans le mélange gazeux et/ou la proportion du gaz de synthèse dans le mélange de gaz de synthèse,
à la condition de ne pas dépasser pendant la totalité du démarrage un taux de conversion maximal des oléfines en C2 à C8 utilisées de 40 à 90 %.

14. Procédé selon la revendication 13, dans lequel on ajoute au mélange d'entrée et/ou au mélange de gaz de synthèse un gaz inerte pour diminuer la proportion des oléfines en C2 à C8 et/ou pour diminuer la proportion de gaz de synthèse, et on réduit l'addition du gaz de synthèse en fonction de l'augmentation pas à pas de la proportion des oléfines en C2 à C8 et/ou de la proportion de gaz de synthèse.
